(19) Europäisches Patentamt
European Patent Office
Office européen des brevets

(11) Veröffentlichungsnummer: **0 329 994 B1**

(12) # EUROPÄISCHE PATENTSCHRIFT

(45) Veröffentlichungstag der Patentschrift: **29.12.93**

(51) Int. Cl.[5]: **C07K 15/14**, C07K 3/02, C07K 15/00, G01N 33/72, C12N 5/00, //C12N15/00, C12P21/00,C07K9/00

(21) Anmeldenummer: **89101835.0**

(22) Anmeldetag: **02.02.89**

Teilanmeldung 93101522.6 eingereicht am 02/02/89.

Die Akte enthält technische Angaben, die nach dem Eingang der Anmeldung eingereicht wurden und die nicht in dieser Patentschrift enthalten sind.

(54) **Immunogen und seine Verwendung zur Gewinnung von Antikörpern gegen Hämoglobin A1C.**

(30) Priorität: **04.02.88 DE 3803330**
**26.02.88 DE 3806198**

(43) Veröffentlichungstag der Anmeldung:
**30.08.89 Patentblatt 89/35**

(45) Bekanntmachung des Hinweises auf die Patenterteilung:
**29.12.93 Patentblatt 93/52**

(84) Benannte Vertragsstaaten:
**AT BE CH DE ES FR GB GR IT LI LU NL SE**

(56) Entgegenhaltungen:
**EP-A- 0 111 211**
**EP-A- 0 185 870**
**EP-A- 0 215 401**
**DE-A- 3 439 610**
**GB-A- 2 024 829**

(73) Patentinhaber: **BOEHRINGER MANNHEIM GMBH**

**D-68298 Mannheim(DE)**

(72) Erfinder: **Klein, Christian, Dr.rer.nat.**
**Blütenstrasse 16**
**D-8120 Weilheim(DE)**
Erfinder: **Hübner-Parajsz, Christa, Dr.rer.nat.**
**Marienstrasse 11**
**D-8132 Tutzing(DE)**
Erfinder: **Batz, Hans-Georg, Dr.rer.nat.**
**Traubinger-Strasse 63**
**D-8132 Tutzing(DE)**
Erfinder: **Rollinger, Wolfgang, Dr.rer.nat.**
**Bärenmühlweg 98**
**D-8120 Weilheim(DE)**
Erfinder: **Essig, Ulrich, Dr.rer.nat.**
**Hofmarkstrasse 22**
**D-8033 Planegg(DE)**
Erfinder: **Kerscher, Lorenz, Dr.rer.nat.**
**Paul Loebe-Strasse 21**
**D-8122 Penzberg(DE)**

EP 0 329 994 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08**
**20**
**D-81635 München (DE)**

## Beschreibung

Die Erfindung betrifft ein Immunogen zur Gewinnung $HbA_{1c}$-spezifischer Antikörper sowie ein Verfahren zur Gewinnung dieser Antikörper.

Hämoglobin, das den Transport von eingeatmetem Sauerstoff und $CO_2$ bewirkt und in den roten Blutkörperchen lokalisiert ist, besteht aus vier Ketten, von denen jeweils zwei die gleiche Struktur haben. Überwiegend besteht es aus zwei alpha-Ketten und zwei $\beta$-Ketten. Das Hämoglobin liegt im Blut zu mehr als 90% in dieser als $HbA_0$ bezeichneten Form vor.

Glycosyliertes Hämoglobin entsteht in vivo durch nichtenzymatische Reaktion von Hämoglobin mit Glucose. Die Glycosylierung verläuft über die Bildung einer Schiffschen Base zwischen der Aldehydgruppe der Glucose und der Aminogruppe des Hämoglobins. Das gebildete Aldimin lagert sich durch eine Amadori-Umlagerung zum N-(1-Desoxy-D-fructos-1-yl)-Rest um. In dieser umgelagerten Form ist das glycosylierte Hämoglobin stabil.

Die glykosylierten Hämoglobine werden mit $HbA_1$ und das wichtigste aus dieser Gruppe mit $HbA_{1c}$ bezeichnet. $HbA_{1c}$ entsteht durch Glycosylierung der freien Aminogruppe des Valin-Restes, der sich am Aminoende der $\beta$-Kette des Hämoglobins befindet. Dabei entsteht ein N-(1-Desoxy-D-fructos-1-yl)-L-Valin-Rest, im folgenden "Fructose Valin" genannt.

Die Konzentration von $HbA_{1c}$ im Blut ist abhängig von der Zuckerkonzentration des Blutes. Der Anteil des $HbA_1$ am Gesamthämoglobin liegt bei Erwachsenen normalerweise im Bereich von 3 bis 6%. Bei erhöhtem Blutzuckerspiegel nimmt der Anteil der glykosylierten Hämoglobine am Gesamthämoglobin zu und kann bis zu 15% ansteigen. Die Bestimmung des $HbA_{1c}$-Anteils ist daher ein zuverlässiger Parameter zur Kontrolle des Zuckerstoffwechsels. Da ein Erythrozyt und mit ihm das stabile $HbA_1$ im Durchschnitt 120 Tage alt wird, bietet somit die Bestimmung des Anteils von glykosylierten Hämoglobinen im Blut einen guten Parameter, um den Kohlehydratstoffwechsel zu kontrollieren, was insbesondere bei Diabetespatienten wichtig ist. Dieser Parameter ist unabhängig von einer kurzzeitigen Erhöhung des Blutzuckerspiegels nach einer kohlehydratreichen Mahlzeit und dient daher als Langzeitparameter.

Es ist daher zur Diagnose der Diabetes und zur Überwachung von Diabetespatienten wichtig, spezifisch den Anteil von $HbA_{1c}$ im Blut zu bestimmen.

Für die Analyse der glykosilierten Hämoglobine gibt es eine Reihe von Verfahren. Die geläufigsten Methoden beruhen auf dem Verlust von positiven Ladungen im Hämoglobin-Molekül, wenn die freien Aminogruppen der $\beta$-Ketten mit Glucose umgesetzt werden. Vor allem säulenchromatographische und elektrophoretische Verfahren finden Verwendung. Andere Methoden versuchen die eingebauten Glucosemoleküle bzw. nach der Amadori-Umlagerung die Fructose-Moleküle colorimetrisch zu erfassen (Thiobarbitursäure-Methode).

Die vorbekannten Verfahren sind zum Teil sehr zeitaufwendig und umständlich und zum Teil auch nicht spezifisch genug für das glykosilierte Hämoglobin.

Es bestand daher Bedarf nach einfachen Bestimmungsverfahren, die sehr spezifisch diesen glycolisierten Anteil im Gesamthämoglobin erfassen.

Solche einfache Verfahren sind die dem Fachmann bekannten verschiedenen Varianten von Immunoassays. Bei homogenen kompetetiven Immunoassays beispielsweise konkurrieren ein markiertes $HbA_{1c}$-Derivat mit $HbA_{1c}$ aus der zu messenden Probe um den Antikörper. Als markiertes $HbA_{1c}$- Derivat kann ein kurzes synthetisches Peptid, das ein Epitop des $HbA_{1c}$ darstellt, verwendet werden und an eine Markierung gebunden werden. Um nun die Bestimmung reproduzierbar mit genauen Ergebnissen durchführen zu können, muß ein Antikörper verwendet werden, der das markierte Peptid und das $HbA_{1c}$ aus der Probe mit annähernd gleich hoher Affinität bindet, um tatsächlich eine Konkurrenzreaktion zu ermöglichen. Zur Durchführung der Immunoassays müssen daher Antikörper bereitgestellt werden, die sehr spezifisch $HbA_{1c}$ erkennen, d.h. spezifisch das glycosylierte N-terminale Ende der $HbA_{1c}$-$\beta$-Kette binden, nicht aber das entsprechende nicht-glycosylierte N-terminale Ende der $HbA_0$-$\beta$-Kette binden.

Eine weitere Variante des kompetitiven Immunoassays wird in heterogener Phase durchgeführt. Dabei konkurriert beispielsweise ein festphasengebundenes synthetisiertes Peptid, das ein $HbA_{1c}$-Epitop enthält, mit dem $HbA_{1c}$ aus der Probe um den spezifischen Antikörper. Das festphasengebundene Peptid kann beispielsweise ein Konjugat aus Rinderserumalbumin und einem synthetischen glycosylierten Peptid sein, das die Sequenz eines Epitops des $HbA_{1c}$ aufweist. Auch für die Durchführung dieser Variante sollte der Antikörper das Peptid und $HbA_{1c}$ aus der Probe mit annähernd gleichhoher Affinität binden.

Um nun ein empfindliches und spezifisches Verfahren zum Nachweis von $HbA_{1c}$ durchführen zu können, war es wesentlich, einen Antikörper zu gewinnen, der spezifisch $HbA_{1c}$, nicht aber $HbA_0$ bindet.

Es sind bereits Verfahren zum Nachweis von $HbA_{1c}$ bekannt, ebenso wie für das Verfahren geeignete Antikörper, z.B. aus EP-A 185 870. Alle bekannten Antikörper leiden jedoch unter dem Nachteil, daß sie

eine sehr geringe Affinität für das $HbA_{1c}$-Molekül aufweisen. In der Regel muß deshalb vor Durchführung einer Bestimmung eine Denaturierung durchgeführt werden, um die antigene Determinante von $HbA_1$ so weit freizulegen, daß die Antikörper in ausreichendem Maße gebunden werden können. Ein derartiges Verfahren ist umständlich.

Die bei dem Verfahren gemäß EP-A 185 870 verwendeten Immunogene haben darüber hinaus den Nachteil, daß sie nur eine wenig spezifische Immunantwort hervorrufen. So konnten nach dem dort beschriebenen Verfahren nur polyklonalen Schaf- und Mausseren gewonnen werden, die keine meßbare Spezifität in Bezug auf die Unterscheidung von $HbA_{1c}$ und $HbA_0$ haben.

Aus DE-OS 34 39 610 ist ein Verfahren zur Gewinnung von Antikörpern gegen $HbA_{1c}$ bekannt, bei dem als Immunogen ein Konjugat aus einem Zucker, einem Peptidrest der $\beta$-Kette des Hämoglobins und einem immunogenen Träger verwendet wird. Die mit diesem Verfahren erhaltenen Antikörper zeigen jedoch keine ausreichende Selektivität und ihre relative Affinität ist gering.

Es war daher Aufgabe der Erfindung, ausgehend von diesem Stand der Technik, ein Verfahren zur Verfügung zu stellen, mit dem hochspezifische polykonale und monoklonale Antikörper gegen $HbA_{1c}$ mit hoher Affinität gebildet werden.

Eine weitere Aufgabe war die Bereitstellung von Antikörpern, die gegenüber dem nativen $HbA_{1c}$-Molekül und einem Peptid, das die N-terminale Sequenz der $\beta$-Kette des Hämoglobins aufweist, molare relative Affinitäten aufweisen, die sich um einen möglichst geringen Faktor unterscheiden.

Diese Aufgabe wird gelöst durch ein Immunogen zur Herstellung $HbA_{1c}$-spezifischer Antikörper mit der allgemeinen Formel I:

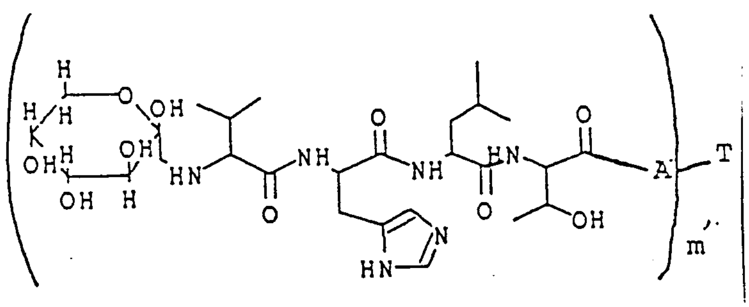

worin m 1 bis zu einer Zahl, die 25 % des Gewichtes des Trägerproteins an Haptenspacergruppen entspricht, bedeutet, T ein Trägerprotein bedeutet und A einen Spacer darstellt, der eine Kettenlänge von 10 bis 20 Atomen aufweist mit der folgenden Formel II.

$$-HN-\underset{\displaystyle COOH}{\underset{|}{CH}}-(CH_2)_n-X-B-$$

worin X S oder NH bedeutet, n 1 bis 4 bedeutet und B einen organischen Rest bedeutet, der eine Succinimidgruppe enthält.

Überraschenderweise wurde festgestellt, daß bei Verwendung der erfindungsgemäßen Immunogene hochspezifische Antikörper erhalten werden können, deren Selektivität und Affinität die der bisher bekannten $HbA_{1c}$-Antikörper bei weitem übertrifft.

Das erfindungsgemäße Immunogen besteht aus drei Teilen - einem Haptenteil, der dem N-terminalen Ende des $HbA_{1c}$-Proteins entspricht, einem Spacer und einem immunogenen Protein. Der Haptenteil des erfindungsgemäßen Immunogens enthält die ersten 4 Aminosäuren des N-terminalen Endes der $\beta$-Kette des Hämoglobins und weiterhin ein Fructosemolekül. Wie in dem nativen $HbA_1$-Protein sind bei dem erfindungsgemäßen Immunogen an das $C_1$-Atom des Fructosemoleküls die Aminosäuren Valin, Histidin, Leucin und Threonin gebunden.

Die Herstellung des Haptenteils erfolgt in an sich bekannter Weise. Als besonders geeignet hat sich die Festphasensynthese (Übersicht in G. Barany und R.W. Merrifield in Gross, Meienhofer, The Peptides, Vol.2, S. 3 - 285, New York, 1978) erwiesen. Dazu werden zweckmäßigerweise die $\alpha$-Aminogruppen mit $N\alpha$-Fluorenylmethoxycarbonyl-Gruppen geschützt. Die Seitenkettenfunktionen werden als tert.-Butylether, tert.-Butylester bzw. als tert.-Butoxycarbonyl-Gruppe geschützt. Diese Schutzgruppen werden bei der acidolytischen Spaltung des fertig synthetisierten Peptids vom Träger mitentfernt. Der N-(1-Desoxy-D-fructos-1-yl)-Rest wird in an sich bekannter Weise durch Reaktion des Peptids mit Glucose eingeführt.

Durch anschließende Amadori-Umlagerung (vgl. K. Heyns und H.Paulsen, J. Liebigs Ann. Chem. 627 - (1959), S. 160 - 174 und H. Röper et al., Carbohydrates. 116 (1983), S. 183 - 195) erhält man dann das gewünschte N-(1-desoxy-D-fructos-1-yl)-Peptid.

Ein weiterer erfindungswesentlicher Bestandteil des immunogenen Konjugats ist der Spacer, der das Hapten mit dem immunogenen Protein verbindet. Der Spacer weist erfindungsgemäß eine Kettenlänge von 10 bis 20 Atomen auf, wobei die Kette aus Kohlenstoff-, Sauerstoff-, Stickstoff- und/oder Schwefelatomen gebildet werden kann. Für die Kettenlänge werden nur die die Kette bildenden Atome gezählt, nicht jedoch die Wasserstoffatome oder die Seitengruppenatome.

Der Spacer wird an die Carboxylgruppe des Threonins gebunden.

Der Spacer weist die folgende allgemeine Formel auf:

$$-HN-\underset{\displaystyle COOH}{\underset{|}{CH}}-(CH_2)_n-X-B-$$

worin X S oder NH bedeutet und n eine ganze Zahl von 1 bis 4 bedeutet. Bevorzugt hat X die Bedeutung S und n 1 bzw. X NH und n 4.

Die Gruppe B der allgemeinen Formel steht für an sich bekannte Spacermoleküle, die eine Succinimidylgruppe aufweisen. Der Aufbau der Gruppe B ist an sich nicht kritisch, sie muß jedoch so viele Kettenmoleküle aufweisen, daß sich zusammen mit dem übrigen Spacerteil eine Kettenlänge von 10 bis 20 Atomen ergibt. Bevorzugt wird als Gruppe B eine Succinimidylhexanoylgruppe verwendet.

Der Spacer wird in an sich bekannter Weise eingeführt. Er enthält eine Aminosäure, die gezielt an das C-terminale Ende des Peptids gebunden werden kann und die eine $NH_2$- oder SH-Gruppe trägt. Über diese Amino- bzw. Mercaptogruppe kann die die Bindung mit dem Trägerprotein vermittelnde Succinimidylgruppe eingeführt werden. Bevorzugt wird als Aminosäure Cystein, Homocystein, Lysin oder Ornithin verwendet. Enthält der Spacer als Aminosäure Cystein oder Homocystein, so kann man zuerst Cystein oder Homocystein mit geschützter Mercaptogruppe, wobei als Schutzgruppe bevorzugt die tert.-Butylsulfenylgruppe eingesetzt wird, als Startaminosäure für die Festphasensynthese des Peptids verwenden. Anschließend setzt man das Trägerprotein mit einem bifunktionellen Linker, der die Succinimidylgruppe liefert, wie z.B. Maleimidohexanoyl-N-hydroxysuccinimid um und kuppelt dann das Spacer-Trägerprotein-Konjugat an die freigesetzte SH-Gruppe des Cysteins bzw. Homocysteins. Enthält der Spacer als Aminosäure Lysin oder Ornithin, so setzt man bevorzugt zuerst die Aminosäure mit geschützter $\alpha$-Aminogruppe als Startaminosäure für die Festphasensynthese des Peptids ein, wobei als Schutzgruppe bevorzugt die Carbobenzoxygruppe verwendet wird und setzt anschließend die freigesetzte $\alpha$-Aminogruppe des Peptids mit dem bifunktionelle Linker um. Das Peptid-Aminosäure-Spacer-Konjugat wird dann an das Trägerprotein gekuppelt, wobei die Bindung über SH-Gruppen des Trägerproteins erfolgt.

Als Trägerprotein können die an sich bekannten Proteine verwendet werden. Geeignet sind beispielsweise Albumine wie Rinderserumalbumin und Ovalbumin, Hämocyanine wie Keyhole limpet hemocyanine, Polyaminosäuren wie Poly-L-Lys und Poly-L-(Lys:Glu) oder Enzyme wie Galactosidase. Bevorzugt wird als Trägerprotein Edestin oder Galactosidase verwendet.

An das Trägerprotein werden bevorzugt mehrere Hapten-Spacergruppen gebunden. Die Anzahl der gebundenen Gruppen hängt von der Größe des Trägerproteins ab. In der Regel können maximal 25% des Gewichtes des Trägerproteins an Hapten-Spacergruppen gebunden werden. Wird als Protein Edestin verwendet, werden bevorzugt 10 bis 30 Haptenspacergruppen gebunden.

Mit den erfindungsgemäß zur Verfügung gestellten Immunogenen werden hochaktive spezifische Antikörper gegen $HbA_{1c}$ erhalten. Diese Antikörper weisen sehr wenig Kreuzreaktivität mit $HbA_0$ auf.

Ein weiterer Gegenstand der Erfindung ist ein Verfahren zur Gewinnung von gegen $HbA_{1c}$ gerichteten Antikörpern. Dazu wird ein erfindungsgemäßes Immunogen in einen geeigneten Organismus mehrmals injiziert und dann in an sich bekannter Weise der Antikörper gewonnen. Zur Gewinnung von Antikörpern werden in der Regel Säugetiere immunisiert. Geeignet sind z.B. Mäuse, Schafe, Kaninchen, Ratten oder Meerschweinchen. Das Immunogen wird, bevorzugt gelöst in Puffer und unter Zusatz eines üblichen Hilfsstoffes, z.B. dem Freundschen Adjuvanz in das Wirtstier injiziert. Um hohe Antikörpertiter zu erzielen, wird die Injektion in regelmäßigen Abständen, z. B. alle zwei bis vier Wochen, wiederholt. Aus dem Blut der Wirtstiere werden in üblicher Weise die Antiseren gewonnen, die die polyklonalen Antikörper enthalten.

Mit den erfindungsgemäßen Immunogenen können auch hochspezifische monoklonale Antikörper gewonnen werden unter Verwendung des bekannten Hybridisierungsverfahren von G. Köhler und C.

Milstein, das beispielsweise in Nature 266, (1977), S. 495 und Science 208, (1980), S. 692 ff. beschrieben ist. Dazu werden nach Immunisierung des Wirtsorganismus B-Lymphozyten aus der Milz der immunisierten Tiere isoliert, mit Myelomzellen fusioniert und die gebildeten Hybridomazellen kloniert. Von den gebildeten Klonen werden dann die Zellinien isoliert, die Antikörper produzieren, die spezifisch mit $HbA_{1c}$ reagieren und praktisch keine Kreuzreaktion mit anderen Molekülen aufweisen. Die Isolierung dieser Zellinien ist einfach, da ein sehr hoher Anteil der Klone spezifische Antikörper produziert. Diese Zelllinien werden weitergezüchtet und daraus können dann die gewünschten monoklonalen Antikörper gewonnen werden. Die Antikörperaktivitäten werden in an sich bekannter Weise im Serum oder den Hybridomaüberständen bestimmt, üblicherweise unter Verwendung eines Enzym-Immunoassays.

Die erfindungsgemäß erhaltenen Antikörper zeichnen sich durch eine hohe Spezifität und hohe Affinität zu $HbA_{1c}$ aus. Die Kreuzreaktion mit nichtglykosiliertem Hämoglobin und mit anderen in Körperflüssigkeiten vorhandenen Proteinen ist vernachlässigbar gering. Sie eignen sich daher ausgezeichnet zur Verwendung in Verfahren zur Bestimmung von $HbA_{1c}$ in Körperflüssigkeiten.

Besonders geeignete monoklonale Antikörper sind die MAK's 3.609.325, 3.51.56 und 3.230.140. Die entsprechenden Hybridoma-Zellinien wurden unter den Nummern ECACC 87120801, ECACC 88122302 und ECACC 88122301 bei der European Collection of Animal Cell Cultures, Proton Down, GB, hinterlegt.

Die Erfindung wird durch die folgenden Figuren und Beispiele erläutert:

Fig. 1 zeigt eine Standardkurve der Aktivität gegen $HbA_{1c}$ von monoklonalen Anti-$HbA_{1c}$-Antikörpern (MAK 3.609.325)

Fig. 2 zeigt die Titerbestimmung (Doppelbestimmung) für ein gemäß Beispiel 14 erhaltenes Antiserum.

Fig. 3 zeigt ein Diagramm mit den relativen Affinitäten der Antikörper gegenüber Peptiden und Hämoglobin.

## Beispiel 1

Es wurde das Peptid Fructose Val-His-Leu-Thr-Cys-OH synthetisiert. Die Festphasensynthese wurde durchgeführt in einem semi-automatischen Peptidsynthesizer der Firma Labortec, Bubendorf (Schweiz).

Als N $\alpha$-Aminoschutzgruppe wurde die Fmoc-Gruppe (Fluorenylmethoxycarbonylgruppe) verwendet. Eine Beschreibung dieser Peptidsynthesemethode findet sich bei J. Meyenhofer et al., Int. J. Peptid Protein Res. 13, 35-42 (1979).

Die C-terminale Fmoc-Aminosäure wurde, wie bei Meyenhofer beschrieben, an p-Alkyloxybenzylalkohol-Harz (Firma Bachem, Bubendorf, Schweiz) gekoppelt.

Syntheseprotokoll für einen Synthesezyklus:

| Schritt | Zeit | Reagenz/Lösungsmittel |
|---|---|---|
| 1 | 2 x 1 min | DMF (Dimethylformamid) |
| 2 | 1 x 3 min | Piperidin/DMF 1 : 4 |
| 3 | 1 x 7 min | Piperidin/DMF 1 : 4 |
| 4 | 4 x 1/2 min | DMF |
| 5 | 2 x 1/2 min | Isopropanol |
| 6 | Stop | Ninhydrintest |
| 7 | 2 x 1 min | DMF |
| 8 | Stop | Zugabe der nächsten Fmoc-Aminosäure und HOBt (1-Hydroxybenzotriazol) in DMF |
| 9 | 2 min | Schütteln |
| 10 | Stop | Zugabe von DCC (Dicyclohexylcarbodiimid) in DCM (Dichlormethan) |
| 11 | 90 min | Kupplung |
| 12 | 3 x 1 min | DMF |
| 13 | 2 x 1 min | Isopropanol |
| 14 | Stop | Ninhydrintest |

Für die Kopplung gemäß Schritt 8 bis 11 werden die Fmoc Aminosäuren und DCC jeweils in der dreifach molaren Menge, bezogen auf die Beladung des Startharzes, eingesetzt. HoBt wird in der 4,5fachen molaren Menge eingesetzt.

Nach der Kopplung der letzten N-terminalen Fmoc-Aminosäure wird zur Abspaltung der Fmoc Schutzgruppe Schritt 1 bis 5 des Synthesezyklus durchlaufen. Danach wird das Harz in dem 15fachen Volumen Dichlormethan (DCM)/Trifluoressigsäure (TFA) 1:1 zwei Stunden bei Raumtemperatur geschüttelt. Man

filtriert, wäscht das Harz noch zweimal mit DCM/TFA 4:1, vereinigt alle Filtrate und dampft im Vakuum unter Toluolzusatz bei 25 ° C ein. Der Rückstand wird mit Diethylether versetzt. Man filtriert den Feststoff ab und trocknet.

Nach dem oben angegebenen Schema wurde das Peptid Fructose-Val-His-Leu-Thr-Cys(StBu)OH synthetisiert. Als Startharz wurden 10 g Fmoc Cys(StBu)p-Alkoxybenzylalkoholharz beladen mit 0,6 mMol/g eingesetzt. In den Synthesezyklen wurden die folgenden Fmoc-Aminosäuren nacheinander eingesetzt:

1. 6,2 g Fmoc Thr (tBu)
2. 6,4 g Fmoc Leu
3. 11 g Fmoc His (Trt)
4. 6,1 g Fmoc Val.

Die folgenden Abkürzungen werden verwendet:

| | |
|---|---|
| tBu: | Tertiärbutylether |
| Trt: | Trityl |
| StBu: | Tertiärbutylthioether |
| OtBu: | Tertiärbutylester |
| Z: | Benzyloxycarbonyl |
| -Cys(StBu): | Tertiärbutylsulfenylcystein |

Die Rohausbeute nach Abspaltung von Harz waren 3,29 g HVal-His-Leu-Thr-Cys (StBu)OH. DC: (Kieselgel HPTLC Merck, Fließmittel: Ethanol/Eisessig/Wasser 6:2:2) $R_f = 0{,}62$. Nach Ansprühen mit Ninhydrinspray 0,1% (Merck) und Entwicklung bei 120 ° C für 5 Minuten ergab sich eine rotviolette Färbung. Beim Ansprühen mit einer Mischung aus 200 ml 0,4%iger methanolischer Resorcinlösung und 40 ml 5 N Schwefelsäure (Resorcinschwefelsäure) und Entwicklung bei 120 ° C für 5 bis 10 Minuten ergab sich keine Färbung.

1 g des Rohpeptides wurde mit 540 mg Glucose und 50 ml Pyridin/Eisessig versetzt und 5 Tage bei Raumtemperatur gerührt. Dann wurde bei Raumtemperatur im Vakuum eingedampft und anschließend noch dreimal der Rückstand mit 50 ml Wasser aufgenommen und Wiederum bis zur Trockene eingedampft. Der Rückstand wurde in 50 ml Wasser aufgenommen, auf eine Säule mit Dowex 50 WX8 ($H^+$-Form, 50x3,5 cm) aufgezogen und mit Wasser gewaschen, bis die gesamte Glucose eluiert war. Danach wurde das Produkt mit 1 N Ammoniak eluiert und lyophilisiert. Man erhielt 810 mg. Das Lyophilisat wurde in 0,1 M Triethylammoniumacetatpuffer, pH 8,5 aufgenommen und auf eine Säule mit Affigel 601 (Biorad, 5x26cm), mit 2 l 0,1 M Triethylammoniumacetatpuffer, pH 8,5 und danach mit 2 l Wasser gewaschen. Das Produkt wurde dann mit 0,1% Ameisensäure eluiert und lyophilisiert. Das so erhaltene Lyophilisat wurde an Polygosil C18, 5µ (Macherey und Nagel) chromatographiert (Gradient 0,1% TFA in Wasser bis 65% Isopropanol in Wasser, 0,1% TFA). Man erhielt 283 mg Fructose Val-His-Leu-Thr-Cys(StBu)OH. DC: (Kieselgel, Fließmittel wie oben): Rf = 0,58. Anfärbung mit Ninhydrin: bräunlich. Anfärbung mit Resorcin-Schwefelsäure: rotbraun. Fab-MS (Fast Atom Bombardment MS) (positiv): $MH^+ = 822$.

Zur Abspaltung der Cysteinschutzgruppe wurde das oben erhaltene Peptid in 130 ml 0,1 M Kaliumphosphatpuffer, pH 8,5 gelöst, mehrmals entgast und wiederum mit Stickstoff belüftet. Die Lösung wurde dann mit 778 mg Dithiotreitol versetzt und 24 Stunden unter Stickstoff stehengelassen. Anschließend wurde mit HCl auf pH 5 angesäuert und durch Chromatographie an Polygosil C18 (Gradient wie oben) gereinigt. Man erhielt 178 mg Fructose Val-His-Leu-Thr-Cys OH FabMS; positiv: $MH^+ = 734$.

**Beispiel 2**

Nach dem in Beispiel 1 angegebenen Syntheseschema wurde das Peptid Fructose ValHisLeuThrLysOH synthetisiert. Als Startharz wurden 10 mg Fmoc Lys(Z) p-Alkoxybenzylalkohol-Harz, beladen mit 0,48 mmol/g verwendet. In den Synthesecyclen werden die folgenden Fmoc-Aminosäuren nacheinander eingesetzt:

1. 5 g Fmoc Thr (tBu)
2. 5,1 g Fmoc Leu
3. 8,9 g Fmoc His(Trt)
4. 4,9 g Fmoc Val

Rohausbeute nach Abspaltung vom Harz: 3,2 g
DC: (Kieselgel HPTLC, Fließmittel siehe Beispiel 1.3): Rf = 0,58
Das Rohpeptid wurde mit 1,6 g Glucose bei Raumtemperatur in 150 ml Pyridin/Eisessig 1:1 gerührt. Die Reaktionslösung wurde eingedampft, der Rückstand wie in Beispiel 1.3 beschrieben, an Dowex 50WX8 $H^+$ und Affigel 601 gereinigt.

Man erhielt 1,7 g Fructose ValHisLeuThrLys(Z)OH.

DC: (Kieselgel HPTLC, Fließmittel siehe Beispiel 1): Rf = 0,58, Anfärbung mit Schwefelsäure Sprühreagenz: rotbraun.

$^1$H-NMR (300 Mhz, $D_2O$): $\delta$ = 0.85 (d, J = 5.1 Hz, 3H); 0.89 (d, J = 4.9 Hz, 3H); 0.96 (d, J = 7.1 Hz, 3H); 1.04 (d, J = 6.8 Hz, 3H); 1.20 (d, J = 1.20, 3H); 1.3-1.9 (m, 9H); 2.3 (m, 2H); 3.0-3.3 (m, 6H); 3.63-4.1 (m, 5H); 4.1-4.29 (m, 4H); 4.32 (d, J = 5.4 Hz, 1H); 4.45 (m, 1H); 4.48 (m, 2H); 5.09 (s, 2H); 7.32 (s, 1H); 7.4 ("s", 5H); 8.63 ppm (s, 1H).

400 mg des oben erhaltenen Produktes wurden in 50 ml Methanol/Wasser 5:1 mit Palladium-Aktivkohle hydriert. Man filtrierte vom Katalysator, dampfte ein und chromatographierte an Polygosil C 18.

Ausbeute: 300 mg

DC (Kieselgel HPTLC, Fließmittel siehe Beispiel 1): Rf = 0,09. Anfärbung mit Schwefelsäure Sprühreagenz: rotbraun.

**Beispiel 3**

10 mg Fructose ValHisLeuThrLysOH aus Beispiel 2 wurden in 1 ml 0,1 M Kaliumphosphatpuffer pH 7 aufgenommen.

Es wurden 6,2 mg Maleimidohexansäure-N-hydroxysuccinimidester in 2 ml Ethanol zugegeben. Die Reaktionslösung wurde 14 Stunden bei Raumtemperatur gerührt und an Polygosil C 18 gereinigt. Man erhielt nach Lyophilisation der reinen Fraktionen 4,3 mg Fructose ValHisLeuThrLys (Maleimidohexanoyl)OH.

FAB-MS, positiv; $MH^+$: 952

$^1$H-NMR (300 MHz, DMSO ($D_6$)/$CD_3OD$): = 0,83 - 1,06 (m, 12H; Leu-$CH_3$, Val-$CH_3$); 1,04 (d, J = 6,5 Hz, 3H; Thr-$CH_3$); 2,01 (t, J = 6,5 Hz, 2H; MH-CO-$\underline{CH_2}$) und 6,92 ppm (s, 2H; MH-CH = CH).

**Beispiel 4**

5 g Edestin aus Hanfsaat (Roth) wurden in 500 ml 0,1 M Kaliumphosphatpuffer pH 7,0 verrührt und mit einer Lösung von 500 mg Maleimidohexansäure-N-hydroxysuccinimidester in 100 ml Ethanol versetzt. Die Lösung wurde 90 Minuten bei Raumtemperatur gerührt, der Feststoff abgesaugt, zweimal mit je 100 ml Wasser, viermal mit je 100 ml Ethanol und nochmals zweimal mit je 100 ml Wasser gewaschen. Der Feststoff wurde in 150 ml Wasser aufgeschlämmt und lyophilisiert.

Ausbeute: 4,34 g

Maleinimidogruppen/mol Edestin: 17 Die Anzahl der Maleinimidogruppen wird wie folgt bestimmt:

| | |
|---|---|
| Lösung A: | 1 mM Cystein, 0,5 mM EDTA in Wasser |
| Lösung B: | 10 mM 5,5'-Dithiobis(2-nitrobenzoat) (Ellmann's Reagenz) in 0,1 M Kaliumphosphatpuffer, pH 8,0 |
| Lösung C: | 1 M Tris . HCl pH 8,2 |

8 mg Maleimidohexanoyl-Edestin werden in 39 ml 0,05 M Kaliumphosphatpuffer aufgenommen und 15 Minuten im Ultraschallbad behandelt.

Danach gibt man 1 ml Lösung A zu und inkubiert 10 Minuten bei 25 °C.

Mit 3,2 ml Lösung C rührt man 2 Minuten bei 25 °C und gibt dann 200 µl Lösung B zu und inkubiert 10 Minuten bei 37 °C. Bei 25 °C zentrifugiert man die Probe 15 Minuten.

Von dem Überstand mißt man die Extinktion bei 405 nm. Den Blindwert erhält man durch die analoge Verfahrensweise ohne Einsatz der Maleimidohexanoyl-Edestin-Probe. Die Anzahl der Maleimidogruppen in nmol ist

$$\frac{\Delta E [mE] \cdot 43,4}{13,3},$$

wobei ΔE die Extinktionsdifferenz zwischen Blindwert und Probe ist. Die molare Menge an Edestin erhält man mit der genauen Einwaage und der relativen Mol-Masse des Edestins von 310 000:

$$\frac{\text{mg Probe}}{0,31} = \text{nmol}$$

Edestin
250 mg des so erhaltenen Maleimidohexanoyl-Edestin wurden unter Argonatmosphäre mit 20 ml 0,1 M Kaliumphosphatpuffer pH 6,5 versetzt. Es wurden ebenfalls unter Sauerstoffausschluß 34 mg Fructose ValHisLeuThrCysOH, das gemäß Beispiel 1 erhalten wurde, zugegeben und 29 Stunden bei Raumtemperatur gerührt. Die Lösung wurde zentrifugiert, der Niederschlag dreimal mit Wasser gewaschen und jeweils wieder zentrifugiert. Der feste Rückstand wurde in 10 ml Wasser aufgeschlämmt und lyophilisiert. Man erhielt 175 mg des Konjugats aus Fructose ValHisLeuThrCysOH und Maleimidohexanoyl-Edestin, das als Immunogen 1 bezeichnet wird.

Wie oben beschrieben, wurden die noch freien Maleimidogruppen bestimmt. Aus der Differenz zu der ursprünglichen Beladung ergibt sich, daß das Immunogen 14,6 mol Peptid pro Mol Edestin enthält.

**Beispiel 5**

48 mg $\beta$-Galactosidase (EIA-Qualität, Boehringer Mannheim GmbH) wurden unter Argonatomsphäre in 2 ml mit Argon begastem 0,1 M Kaliumphosphatpuffer pH 7,0 gelöst. Dazu gab man unter Sauerstoffausschluß 5 mg Fructose ValHisLeuThrLys(MH)OH, das gemäß Beispiel 3 erhalten wurde und rührte eine Stunde bei Raumtemperatur.

Eine AcA 202-Säule (2x24cm) wurde mit Argon-gesättigter 0,9% NaCl äqulibiriert. Die gesamte Reaktionslösung wurde auf diese Säule aufgetragen. Man eluierte mit Argon-gesättigter 0,9 NaCl und sammelte die Proteinfraktion. Man erhielt 15 ml Immunogen-Lösung, c = 3,1 mg/ml. Die Beladung mit Immunogen kann man durch die Umsetzung einer Probe mit Ellmann's Reagenz bestimmen: pro Mol SH-Gruppe wird 1 Mol Carboxynitrothiopyridon frei ($\lambda_{max}$ = 412 nm, $\epsilon$ = 13.600 bei pH 8,0).

$\beta$-Galactosidase EIA-Qualität enthält 14 SH-Gruppen pro Molekül. Nach Reaktion mit dem Peptid findet man zwei freie SH-Gruppen, d.h. die Beladung ist 12 Mol Peptid pro Mol $\beta$ = Galactosidase.

Das erhaltene Konjugat aus Fructose ValHisLeuThrLys(MH)OH und $\beta$-Galactosidase wird als Immunogen 2 bezeichnet.

**Beispiel 6**

Zum Vergleich wurde ein Immunogen hergestellt, das eine längere Peptidkette aufwies. Dazu wurde erst das Peptid ValHisLeuThrProGluGluCysOH nach dem im Beispiel 1 beschriebenen Syntheseschema hergestellt.

Es wird nach dem Syntheseschema von Beispiel 1 das Peptid HValHisLeuThrProGluGluCys(StBu)OH hergestellt. Als Startharz wird 10 g FmocCys(StBu)p-Alkoxybenzylalkoholharz verwendet mit einer Beladung von 0,5 mmol/g. In den Synthesezyklen werden die folgenden Aminosäuren eingesetzt:

1. 6,4 g Fmoc Glu (OtBu)
2. 6,4 g Fmoc Glu (OtBu)
3. 5,1 g Fmoc Pro
4. 6 g Fmoc Thr (tBu)
5. 5,3 g Fmoc Leu
6. 9,3 g Fmoc His (Trt)
7. 5,1 g Fmoc Val; die Kopplung wurde einmal wiederholt.

Rohausbeute nach Abspaltung vom Harz: 4,2 g. Das Rohprodukt wurde, wie im Beispiel 1 beschrieben, an Polygosil C18 chromatographiert. Die Ausbeute betrug 880 mg HValHisLeuThrProGluGluCys(StBu)OH DC (Kieselgel, Fließmittel wie im Beispiel 1): $R_f$ = 0,53. 800 mg dieses Peptids wurden dann, wie in Beispiel 1 beschrieben mit Glucose umgesetzt und an Dowex 50WX8 $H^+$-Form, danach an Affigel 601 aufgereinigt. Nach Chromatographie an Polygosil C 18 erhielt man 150 mg Produkt. FabMS, positiv: $MH^+$ = 1177. Analog Beispiel 1 wurden dann die Cysteinschutzgruppen abgespalten durch Zugabe von Dithiothreitol. Man erhielt 78 mg Fructose ValHisLeuThrProGluGluCysOH. FabMS positiv: $MH^+$ = 1087.

Das so erhaltene Peptid setzt man mit 322 mg Maleidohexanoyledestin wie im Beispiel 4 beschrieben um. Man erhielt so 200 mg Immunogen, das als Vergleichsimmunogen V1 verwendet wurde.

**Beispiel 7**

Als weiteres Vergleichsimmunogen wurde ein Konjugat aus Fructose ValHisLeuThrCys und Pyridyldithiopropionyledestin hergestellt. Das Peptid wurde hergestellt, wie im Beispiel 2 beschrieben.

Zur Herstellung von Pyridyldithiopropionyledestin wurden 1 g Edestin aus Hanfsaat in 150 ml 0,1 M Kaliumphosphatpuffer, pH 7,5 aufgenommen. Zu dieser Lösung wurden unter Rühren 10 mg Succinimidyl-3(2-pyridyldithio)-propionat, gelöst in 12,5 ml Ethanol, zugegeben. Es wurde zwei Stunden gerührt, dann der Feststoff abgesaugt, mit je dreimal 100 ml Wasser, 100 ml Ethanol und nochmals 100 ml Wasser gewaschen. Der Feststoff wurde in 100 ml Wasser aufgenommen und lyophilisiert.

352 mg des so erhaltenen Pyridyldithiopropionyledestin wurden unter Argonatmosphäre mit 100 ml 0,05 M Kaliumphosphatpuffer, pH 6,0 versetzt. Man gab ebenfalls unter Sauerstoffausschluß 49,5 mg des Peptids Fructose ValHisLeuThrCys zu und rührte 23 Stunden bei Raumtemperatur. Anschließend wurde zentrifugiert, der Niederschlag dreimal mit je 50 ml Wasser, zweimal mit je 50 ml Ethanol und noch einmal mit Wasser gewaschen und jeweils wieder abzentrifugiert. Der feste Rückstand wurde in 50 ml Wasser aufgenommen und lyophilisiert. Zur Bestimmung der Beladung wurde das freigesetzte Thiopyridon im Überstand der ersten Zentrifugation gemessen. Man erhielt 300 mg des als Vergleichsimmunogen V2 bezeichneten Konjugats aus Fructose ValHisLeuThrCys und Pyridyldithiopropionyledestin. Die Beladung betrug 39 Mol Peptid pro Mol Edestin.

**Beispiel 8**

Zur Durchführung des Screeningtests auf Anti-$HbA_{1c}$-Antikörper werden Polyhaptene hergestellt. Polyhapten 1 wird aus HValHisLeuThrProGluGluCysOH und Pyridyldithiopropionyl-Rinderserumalbumin hergestellt.

1 g Rinderserumalbumin wurde in 30 ml 0,1 M Kaliumphosphatpuffer pH 7,5 gelöst. Zu dieser Lösung wurde 226 mg Succinimidylpyridyldithiopropionat gelöst in 15 ml Ethanol gegeben. Man rührte 40 Minuten bei Raumtemperatur und chromatographierte die gesamte Reaktionslösung über ACA 202 (31x3cm); Eluat 0,1 M Kaliumphosphatpuffer pH 6,0. Man erhielt 152 ml Proteinlösung, c = 6,6 mg/ml. Die Lösung wurde mit 280,5 mg Dithiothreitol versetzt und füllt mit 0,1 M Kaliumphosphatpuffer pH 7,5 auf 10 ml aufgefüllt. Man verdünnte um den Faktor 6. Die Konzentration des freigesetzten Thiopyridons entsprach der Konzentration der eingesetzten Dithiopyridylgruppen. Aus dem Extinktionskoeffizienten für Thiopyridon bei 340 nm von 8080 und der relativen Molmasse von Edestin von 310.000 kann die Beladung bestimmt werden. Sie ist 36 Mol/Mol Protein.

10,5 ml der erhaltenen Lösung aus Pyridyldithiopropionyl-Rinderserumalbumin und 47 mg HValHisLeuThrProGluGluCysOH, das gemäß Beispiel 6 erhalten wurde, wurden unter Argon (Sauerstoffausschluß) einen Tag gerührt. Die Beladung wurde durch Bestimmung des freigesetzten Thiopyridons berechnet mit von 33 Mol Peptid/Mol Protein. Restliche reaktive Gruppen werden durch Zugabe von 1,7 mg Cystein abgesättigt. Die Gesamtreaktionslösung wird über eine ACA 202-Säule (31x3 cm) chromatographiert, die Proteinfraktion über Nacht gegen $H_2O$ dialysiert und lyophilisiert. Das erhaltene Produkt wird als Polyhapten 1 bezeichnet.

Ausbeute: 85 mg.

**Beispiel 9**

Zur Herstellung des Polyhaptens 2 (vgl. Beispiel 8) verwendet man 7 ml Pyridyldithiopropionyl-Rinderserumalbuminlösung, die wie im Beispiel 6 beschrieben, erhalten wurden. Als Peptidkomponente werden 7,8 mg des gemäß Beispiel 6 hergestellten Peptids, das an der Aminosäure Val eine Fructose trägt, verwendet. Dazu werden 800 mg des gemäß Beispiel 6 erhaltenen Peptids HValHisLeuThrProGluGluCysOH wie im Beispiel 1 beschrieben, mit Glucose umgesetzt und an Dowex 50 WX H-Form, danach an Affigel 600 aufgereinigt. Nach Chromatographie an Polygosil C18 erhielt man 150 mg des glykosylierten Peptides. FabMS positiv, $MH^+ = 1177$. Nach Abspaltung der Cysteinschutzgruppe mit Dithiothreitol erhielt man aus 100 mg des glycosylierten Peptides 78 mg des gewünschten Produktes. FabMS positiv $MH^+ = 1089$. Das so erhaltene Produkt wird als Polyhapten 2 bezeichnet.

**Beispiel 10**

Um die Anwesenheit und Spezifität von Antikörpern gegen $HbA_{1c}$ im Serum immunisierter Mäuse oder in dem Kulturüberstand der Hybridzellen oder in Ascites zu erkennen, wurde ein Elisa-Verfahren als Testprinzip verwendet:

Mikrotiterplatten wurden mit 10 µg Polyhapten 2 oder 10 µg Polyhapten 1/ml Beschichtungspuffer (0,2 M Natriumcarbonat/-bicarbonat, pH 9,3 bis 9,5) bei Raumtemperatur eine Stunde lang unter Schütteln beschichtet. Dann wurde 10 Minuten mit 0,9% Natriumchloridlösung und 1% Albuminlösung nachbehandelt. Anschließend wurde mit 0,9% Natriumchloridlösung gewaschen. Danach wurde bei Raumtemperatur etwa eine Stunde mit 100 µl Probe inkubiert und erneut mit 0,9% Natriumchloridlösung gewaschen. Es folgte

eine weitere Inkubation für eine Stunde mit 200 U/ml eines Schaf-Fab-Anti-Maus-Fcγ-Peroxidase-Konjugats. Nach einem erneuten Waschschritt mit 0,9% Natriumchlorid wird die Peroxidaseaktivität in üblicher Weise bestimmt, indem man 15 Minuten bei Raumtemperatur mit ABTS umsetzt. Danach bestimmt man die Extinktionsdifferenz, Unterschied mE, bei 405 nm.

Die Antiseren, die erhalten wurden nach Immunisierung mit dem erfindungsgemäßen, gemäß Beispiel 4 erhaltenem Immunogen 1 ergaben bei allen 100 immunisierten Mäusen eine Bindung mit Polyhapten 2 und keine bzw. nur eine schwache Bindung mit Polyhapten 1. Es wurden also selektiv mit Polyhapten 2 bindende Antikörper erhalten.

Von den Antiseren, die nach Immunisierung mit dem erfindungsgemäßen, gemäß Beispiel 6 hergestellten Immunogen 2 erhalten wurden, reagierten 6 von 18 aus immunisierten Mäusen erhaltenen Seren mit Polyhapten 2 und nicht mit Polyhapten 1. Die Bindung an Polyhapten 2 war dabei ebenso gut wie beim Immunogen 1. Auch hier wurden also selektive Antikörper erhalten.

Bei der zum Vergleich durchgeführten Immunisierung mit dem Vergleichsimmunogen V1 wurde von 100 Mäusen ein Antiserum erhalten, das präferentiell mit Polyhapten 2 reagierte, wobei alle anderen 99 Antiseren gleich gut mit beiden Polyhaptenen reagierten. Hier konnten also keine selektiv mit Polyhapten 2 bindenden Antikörper erhalten werden.

Bei der Immunisierung mit dem Vergleichsimmunogen V2 konnten keine differenzierenden Maus-Antiseren erhalten werden. Alle von 100 Mäusen erhaltenen Antiseren reagierten mit beiden Polyhaptenen gleich gut. Dabei war die Bindung an die Polyhaptene um den Faktor 10 schwächer als bei den mit den erfindungsgemäßen Immunogenen 1 und 2 erhaltenen Seren.

**Beispiel 11**

Balb/c- und B10.D2-Mäuse, 8 bis 12 Wochen alt, wurden mit 100 µg $HbA_{1c}$ (β 1-4 Cys, MHS)-Edestin (Immunogen 1) in komplettem Freund'schem Adjuvans (CFA) intraperitoneal (i.p.) erstimmunisiert. Nach 6 Wochen und 10 Wochen wurden zwei weitere Immunisierungen durchgeführt. Dabei wurden 100 µg Immunogen in inkomplettem Freund'schem Adjuvans (IFA) verabreicht. Zehn Tage nach der letzten Immunisierung wurden die Mäuse geblutet, um aus dem Serum den Antikörpertiter zu bestimmen. 4 Tage und 3 Tage vor der Fusion wurden die Mäuse nochmals mit je 100 µg Immunogen in Puffer intravenös immunisiert.

Zur Fusion werden in Anlehnung an Galfré, Methods in Enzymology, 73 (1981), Seite 3, einmal $10^8$ Milzzellen einer immunisierten Maus mit $2x10^7$ Myelomzellen (P3x63Ag8-653, ATCC-CRL 8375) gemischt und anschließend 10 Minuten zentrifugiert (300 g, 4°C). Die Zellen werden noch einmal mit BSS (= balanced salt solution) gewaschen und bei 400 g in einem 50 ml Spitzröhrchen zentrifugiert. Der Überstand wurde entfernt. Das Zellsediment wurde aufgelockert und mit 1 ml 60%iger PEG-Lösung (MG 4000, Merck) versetzt. Nach einer Minute im Wasserbad wurde bei Raumtemperatur 5 ml RPMI 1640-Medium (RPM = Rosewell Parker Memory Institut) ohne fötales Kälberserum (FKS) in einem Zeitraum von 4 bis 5 Minuten zugetropft, durchgemischt, mit Medium auf 50 ml aufgefüllt und anschließend 10 Minuten bei 400 g, 4°C zentrifugiert. Die sedimentierten Zellen wurden in RPMI 1640-Medium mit 10% FKS aufgenommen. Je $10^5$ Milzzellen werden auf 24-well-Zellkulturplatten (Firma Nunc) eingesät. Zu jeder Kultur wurden $5x10^4$ Peritoneal-Exsudat-Zellen als Futterzellen zugefügt. Am folgenden Tag wurde Hypoxanthin-Azaserin-Selektionsmedium (100 mM Hypoxanthin, 1 µg/ml Azaserin) zugegeben.

Nach ca. 7 bis 10 Tagen waren bereits viele Klone sichtbar. Der Überstand der Primärkulturen wurde nach einem in Beispiel 10 beschriebenen ELISA-Verfahren getestet. Primärkulturen, die nur eine geringe oder keine Kreuzreaktion mit $HbA_0$ zeigten, wurden mit Hilfe eines fluoreszenzaktivierenden Zellsorters (FACS) auf 96-well-Zellkulturplatten (Firma Nunc) weiter kloniert. Als Futterzellen dienten $1x10^4$ Peritoneal-Exsudat-Zellen pro well.

Auf diese Weise konnten beispielsweise die Hybridoma-Zellinien isoliert werden, die bei der European Collection of Animal Cell Cultures unter den Hinterlegungsnummern (1.) ECACC 87120801, (2.) ECACC 88122302 und (3.) ECACC 88122301 hinterlegt worden sind. Aus diesen Zellinien können die monoklonalen Antikörper (1.) 3.609.325, (2.) 3.51.56 und (3.) 3.230.140 erhalten werden.

Zur Erzeugung von Ascites wurden Mäuse mit $5x10^6$ Hybrid-Zellen intraperitoneal gespritzt, die zuvor ein- bis zweimal mit 0,5 ml Pristan vorbehandelt worden waren. Eine bis drei Wochen danach konnte aus den Mäusen Ascites-Flüssigkeit mit einer IgG-Konzentration von 5 bis 20 mg/ml gewonnen werden. Hieraus konnten in üblicher Weise die Antikörper isoliert werden. Diese monoklonalen Antikörper sind spezifisch gegen $HbA_{1c}$ gerichtet und zeigen keine bzw. nur eine geringe Kreuzreaktivität mit $HbA_0$.

**Beispiel 12**

Materialien

| | |
|---|---|
| Mikrotiterplatten: | A: NUNC 4-42404 II B: NUNC 2-69620 |
| 12-Kanal-Pipette: | Dynatech, Katalog-Nr. 77-887-00 |
| Plattenschüttler: | Flow Laboratories, Titertek, Katalog-Nr. 77-471-00 |
| Abdeckfolien: | Dynatech Plate Sealers, Katalog-Nr. M 30 |
| ELISA-Reader: | Dynatech MR 700 |
| Beschichtungspuffer: | 50 mM Natriumcarbonat, pH 9,6 |
| Probenpuffer: | 10 mM Natriumphosphat, pH 7,4, 0,9% NaCl, 0,1% Tween 20, 1% Crotein C |
| Waschpuffer: | 0,9% NaCl, 0,1% Tween 20 |
| Antikörper-Enzym-Konjugat: | Konjugat aus Peroxidase und dem Fab-Fragment eines polyklonalen Antikörpers aus Schaf, der gegen den Fcγ-Teil von Maus-IgG gerichtet ist, 25 mU/ml in Probenpuffer |
| Substrat: | 100 mmol/l Phosphat-Citrat-Puffer pH 4,4 3,2 mmol/l Natriumperborat 1,9 mmol/l ABTS (2,2'-Azino-di-[3ethylbenzthiazolin-sulfonsäure(6)] diammoniumsalz) |
| Antikörper: | MAK 3.609.325 (ECACC 87120801) |
| Polyhaptene: | Polyhapten 1 gemäß Beispiel 8 Polyhapten 2 gemäß Beispiel 9 |
| Antigen: | $HbA_{1c}$ nativ<br>$HbA_0$ nativ |

In einem Vorversuch wurden die in die eigentlichen Spezifitätsexperimente einzusetzenden Antikörpermengen bestimmt.

Hierzu wurden Mikrotiterplatten mit Polyhapten 1 bzw. Polyhapten 2 beschichtet. Je 100 µl Napf wurden 1 µg Polyhapten pro ml Beschichtungspuffer eine Stunde lang bei Raumtemperatur inkubiert. Danach wurde die Lösung abgesaugt und dreimal mit Waschpuffer gewaschen.

Anschließend wurden dem festphasengebundenen Polyhapten Verdünnungsreihen des Antikörpers 3.609.325 (Ascites) zugegeben, wobei die Verdünnung mit Probenpuffer ab 1:100 in Viererschritten erfolgte. Es wurde jeweils mit 100 µl/Napf eine Stunde bei Raumtemperatur inkubiert und anschliessend gewaschen.

Die an das Polyhapten gebundenen Antikörper wurden durch Zugabe eines Konjugats aus Peroxidase und dem Fab-Fragment eines polyklonalen Antikörpers aus Schaf, der gegen den FCγ-Teil von Maus-IgG gerichtet ist, über die Reaktion der Peroxidase mit zugegebenem Substrat bestimmt. Es wurden 100 µl Konjugat/Napf zugegeben und eine Stunde bei Raumtemperatur inkubiert. Die Nachweisreaktion wurde durch Zugabe von 100 µl Substrat/Napf in alle Näpfe gestartet. Die Messung erfolgt im ELISA-Reader bei 405 nm (Referenzwellenlänge 490 nm).

Als Titer wurde diejenige Antikörperverdünnung definiert, bei der eine halbmaximale Bindung stattfindet. Diese Antikörpermenge wurde in die folgenden Versuche eingesetzt.

Die Spezifität der monoklonalen Antikörper wurde untersucht. Dazu wurden die Reaktivitäten einzelner Antikörper mit verschiedenen in Lösung befindlichen Komponenten verglichen.

In mit 1% Crotein C vorbeschichtete Mikrotiterplatten wurden je 50 µl einer Lösung des monoklonalen Antikörpers in doppelter Titerkonzentration und 50 µl Antigen-Lösung (Verdünnungsreihe, siehe unten) pipettiert, und gemischt und 30 Minuten bei Raumtemperatur inkubiert. Danach wurden 100 µl Aliquots der Gemische in die mit Polyhapten beschichteten Mikrotiterplatten transferiert.

Verdünnungsreihen:

| | |
|---|---|
| Fructose ValHisLeuThrCys(Stbu)OH<br>(gemäß Beispiel 1)<br>Fructose ValHisLeuThrProGluGluCys(StBu)OH<br>(gemäß Beispiel 6)<br>Fructose Valin | ab 5 µg/ml Probenpuffer mit Probenpuffer in Viererschritten |

| | |
|---|---|
| HValHisLeuThrProGluGluCys(StBu)OH<br>(gemäß Beispiel 6)<br>$HbA_{1c}$<br>$HbA_0$ | ab 100 µg/ml Probenpuffer in Viererschritten |

Die zur halbmaximalen Bindung gehörige Konzentration in mol/l einer zu bestimmenden Substanz wird als molare relative Affinität definiert.

Zum Vergleich der Reaktivität einers monoklonalen Antikörpers mit verschiedenen Komponenten wird die relative Affinität des monoklonalen Antikörpers für die Komponente FructoseValHisLeuThrProGluLfuCys-(Stbu)OH gleich 100% gesetzt. Die Reaktivitäten, die auch als Kreuzreaktionen bezeichnet werden, mit anderen Komponenten ergeben sich dann aus dem Quotienten der relativen Affinitäten wie folgt:

$$\text{Kreuzreaktion} = \frac{C_{\text{rel. Aff. FructoseValHisLeuThrProGluGluCys(StBu)OH}}^{(nM)}}{C_{\text{rel. Aff. X}}^{(nM)}} \times 100$$

Die für die einzelnen Komponenten erhaltenen Werte ergeben sich aus der folgenden Tabelle 1.

Dabei wurden auch Ergebnisse ausgewertet, die mit verschiedenen, in analoger Weise erhaltenen Antikörpern erhalten wurden.

Die Tabelle zeigt, daß mit den erfindungsgemäßen Imunogenen eine Vielzahl von monoklonalen Antikörpern hergestellt werden kann, die sehr spezifisch $HbA_{1c}$, nicht aber $HbA_0$ binden. Verglichen mit den Antikörpern, die unter Verwendung des Vergleichsimmunogens V1 erhalten wurden oder den aus EPA 185 870 bekannten Antikörpern, erkennen die erfindungsgemäß erhaltenen Antikörper $HbA_{1c}$ ohne besondere Denaturierung mit wesentlich höherer Affinität. Sie eignen sich daher sehr gut für kompetitive Immunoassays.

Tabelle 1

**Spezifitätsuntersuchungen von <HbA$_{1c}$>-Klonen**

Ergebnisse:

| Reaktivität mit ⊕ | KMC 19.2 | KMC 19.3 | MAK 1 3.51.56 | MAK 2 3.230.140 | MAK 3 | MAK 4 | MAK 5 | MAK 3.609.325 | MAK 6 | MAK 7 |
|---|---|---|---|---|---|---|---|---|---|---|
| Polyhapten 2 | + | + | + | + | + | + | + | + | + | + |
| Peptid A | 100 | 100 | 100 | 100 | 100 | 100 | 100 | 100 | 100 | 100 |
| HbA$_{1c}$ nativ | 4,4 | 9,4 | 15,3 | 20,0<br>22,0 | 20,7 | 40,4 | 25,0 | 40,0 | 4,1 | 7,1 |
| Fructose-Valin | < 0,29 | < 0,03 | < 0,01 | n.b. | < 0,56 | < 0,11 | n.b. | n.b. | < 0,04 | < 0,1 |
| Polyhapten 1 | - | - | - | n.b. | - | - | n.b. | n.b. | n.b. | n.b |
| Peptid B | < 0,06 | < 0,01 | < 0,01 | < 0,03 | < 0,1 | < 0,02 | < 0,04 | < 0,24 | 3 | 5,1 |
| HbA$_0$ nativ | < 3,5 | < 0,4 | < 0,13 | < 0,95 | < 6,0 | < 1,3 | < 1,1 | < 6,7 | < 0,5 | < 0,13 |

n.b. = nicht bestimmt

+ Klone mit der Bezeichnung KMC: EPA 185 870

MAK 1 bis MAK 5 und MAK 3.609 325: hergestellt mit Immunogen aus Beispiel 4
MAK 6 und MAK 7, : hergestellt mit Vergleichsimmunogen V1 aus Beispiel 6
Peptid A: Fructose ValHisLeuThrProGluGluCys(StBu)OH
Peptid B: HValHisLeuThrProGluGluCys(StBu)OH

## Beispiel 13

Wie in Beispiel 12 beschrieben, werden in mit 1 % Crotein C vorbeschichtete Microtiterplatten je 50 µl einer Lösung des MAK 3.609.325 in doppelter Titerkonzentration und 50 µl HbA$_{1c}$-Lösung pipettiert,

gemischt und bei Raumtemperatur 30 Min. inkubiert. Eingesetzt werden verschiedene Konzentrationen von $HbA_{1c}$.

Danach werden 100 µl-Aliquots der Gemische in mit Polyhapten 2 beschichtete Mikrotiterplatten überführt. Die an das Polyhapten 2 gebundenen Antikörper werden, wie in Beispiel 12 beschrieben, mit Hilfe eines Konjugats aus Peroxidase und dem Fab-Fragment eines polykonalen Antikörpers aus Schaf, der gegen Fcγ-Teil von Maus-IgG gerichtet ist, nachgewiesen.

Je mehr $HbA_{1c}$ in der Probelösung vorhanden ist, desto weniger Antikörper binden an das Polyhapten, d.h. desto geringer ist die gemessene Extinktion. In Abbildung 1 ist die bei der Bestimmung von $HbA_{1c}$ erhaltene Kurve dargestellt.

**Beispiel 14**

Je 10 Schafe wurden mit den erfindungsgemäßen Immunogenen 1 bzw. 2 in komplettem Freund'schem Adjuvans immunisiert. Die Dosis betrug jeweils 200 µg Immunogen pro Tier für die erste und jede Folgeimmunisierung. Die Immunisierungen erfolgten in monatlichen Abständen.

Die erhaltenen Seren wurden, wie im Beispiel 12 beschrieben, auf Anwesenheit und Spezifität von Antikörpern gegen $HbA_{1c}$ untersucht. Dazu wurden Mikrotiterplatten mit 0,04 µg/ml Polyhapten 2 beschichtet. Als Antikörper-Enzym-Konjugat wurde ein Konjugat aus Peroxydase und einem Kaninchen-Antischaf-Immunglobulin verwendet. Die Einsatzkonzentration dieses Konjugats betrug 150 mU/ml. Als Antigene wurden $HbA_{1c}$ und $HbA_0$ nativ verwendet, als Peptide HValHisLeuThrProGluGluCysOH und Fructose ValHisLeuThrProGluGluCysOH.

Das Verfahren wurde, wie im Beispiel 12 beschrieben, durchgeführt.

Es wurden folgende Proben untersucht:

Pool 1: Mischung aus Aliquots von Serumproben aller 10 Tiere, die mit dem erfindungsgemäßen Immunogen aus Beispiel 4 behandelt worden waren (Probeblutung 45 Tage nach Erstimmunisierung).

Pool 2: Mischung aus Aliquots von Serumproben aller 10 Tiere, die mit dem erfindungsgemäßen Immunogen 2 aus Beispiel 5 behandelt worden waren (Probeblutung 45 Tage nach Erstimmunisierung).

Probe a: Serumprobe von Schaf 3227, das mit dem erfindungsgemäßen Immunogen 1 aus Beispiel 4 behandelt worden war (Probeblutung 165 Tage nach Erstimmunisierung)

Probe b: Serumprobe von Schaf 3233, das mit dem erfindungsgemäßen Immunogen 1 aus Beispiel 4 behandelt worden war (Probeblutung 165 Tage nach Erstimmunisierung)

Probe c: Serumprobe von Schaf 3272, das mit dem erfindungsgemäßen Immunogen 2 aus Beispiel 5 behandelt worden war (Probeblutung 75 Tage nach Erstimmunisierung).

Die Titerbestimmung lieferte die folgenden Ergebnisse:

Pool 1: 1:5.400
Pool 2: 1:8.400
Probe a: 1:3.700
Probe b: 1:3.600
Probe c: 1:2.600

Fig. 2 zeigt ein Beispiel für den Extinktionsverlauf in Abhängigkeit von der Antiserumkonzentration.

**Beispiel 15**

Wie in Beispiel 14 beschrieben, wurden die Affinitäten und Spezifitäten von Antikörpern zu glycosilierten und nicht-glycolisierten Antigenen bestimmt. Die Ergebnisse sind den Tabellen 2 und 3 sowie der Fig. 3 zu entnehmen.

In Fig. 3 bedeuten:

| Kurve | Antigen | maximale Einsatzkonzentration (nmol/l) |
|---|---|---|
| 1 | Peptid A | 5340 |
| 2 | Peptid B | 16000 |
| 3 | $HbA_{1c}$ | 2670 |
| 4 | $HbA_0$ | 2670 |

Die Tabellen zeigen, daß mit den erfindungsgemäßen Immunogenen hochspezifische polyklonale Antikörper hergestellt werden können, die sehr spezifisch $HbA_{1c}$, nicht aber $HbA_0$ binden. Gezeigt wird auch, daß die polyklonalen Antikörper $HbA_{1c}$ ohne Denaturierung mit hoher Affinität erkennen. Weiterhin ist aus den Ergebnissen der verschiedenen Pools, die eine Mischung der von jeweils zehn Schafen nach Behandlung mit den erfindungsgemäßen Immunogenen erhaltenen polyklonalen Antikörpern darstellen, ersichtlich, daß bei Verwendung der erfindungsgemäßen Immunogene ein sehr hoher Prozentsatz der immunisierten Tiere geeignete Antikörper produziert.

Tabelle 2

| Affinität der Antikörper zu glykosylierten und nicht glykosylierten Antigenen | | | | | |
|---|---|---|---|---|---|
| Probe | Relative Affinität (nmol/l) | | | | Kreuzreaktion $HbA_0/HbA_{1c}$ |
| | Peptid A* | Peptid B** | $HbA_{1c}$ | $HbA_0$ | |
| Pool 1 | 21 | ≫16000 | 30 | ≫2670 | ≪ 1,1 % |
| Probe a | 33 | ≫16000 | 115 | ≫2670 | ≪ 4,3 % |
| Pool 2 | 15 | ≫16000 | 18 | ≫2670 | ≪ 0,7 % |
| Probe c | 21 | ≫16000 | 134 | ≫2670 | ≪ 5,0 % |

* Peptid A: Fructose ValHisLeuThrProGluGluCys(StBu)OH
**Peptid B: HValHisLeuThrProGluGluCys(StBu)OH

## Tabelle 3

Spezifitätsuntersuchungen von polyklonalen Antikörpern gegen $HbA_{1c}$

Ergebnisse:

| Reaktivität mit + | Pool1 | Probe a | Pool 2 | Probe c |
|---|---|---|---|---|
| Polyhapten 2 | + | + | + | + |
| Peptid A | 100 | 100 | 100 | 100 |
| $HbA_{1c}$ nativ | 70 | 28,6 | 83,3 | 15,7 |
| Polyhapten 1 | - | - | - | - |
| Peptid B | ≪0,13 | ≪0,2 | ≪0,09 | ≪0,13 |
| $HbA_0$ nativ | ≪0,8 | ≪1,2 | ≪0,5 | ≪0,8 |

\+ Pool 1, Probe a: hergestellt aus Immunogen 1 des Beispiels 4
Pool 2, Probe c: hergestellt aus Immunogen 2 des Beispiels 5

**Patentansprüche**
**Patentansprüche für folgende Vertragsstaaten : AT, BE, CH, DE, FR, GB, IT, LI, LU, NL, SE**

1. Immunogen zur Gewinnung von $HbA_{1c}$-spezifischen Antikörpern mit der allgemeinen Formel I:

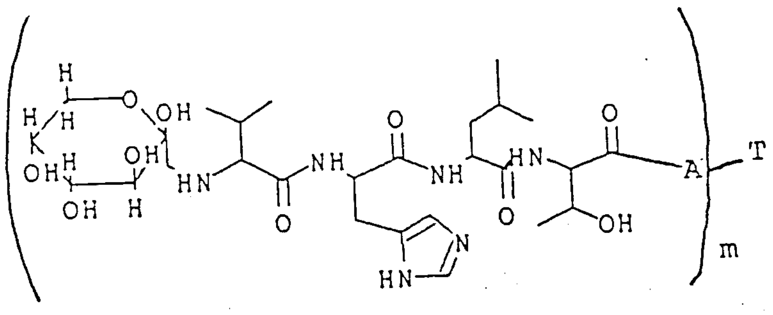

worin m 1 bis zu einer Zahl, die 25 % des Gewichtes des Trägerproteins an Haptenspacergruppen entspricht, bedeutet, T ein Trägerprotein bedeutet und A einen Spacer darstellt, der eine Kettenlänge von 10 bis 20 Atomen aufweist mit der folgenden Formel II.

$$-HN-\underset{\displaystyle COOH}{\underset{|}{CH}}-(CH_2)_n-X-B-$$

worin X S oder NH bedeutet, n 1 bis 4 bedeutet und B einen organischen Rest bedeutet, der eine Succinimidgruppe enthält.

2. Immunogen nach Anspruch 1, **dadurch gekennzeichnet,** daß der Rest A eine Kettenlänge von 12 bis 18 Atomen aufweist.

3. Immunogen nach Anspruch 1 oder 2, **dadurch gekennzeichnet,** daß m 3 bis 20 bedeutet.

4. Immunogen nach einem der vorhergehenden Ansprüche, **dadurch gekennzeichnet,** daß B einen Succinimidyl-Hexanoylrest darstellt.

5. Verfahren zur Gewinnung von Antikörpern, die spezifisch glykosyliertes Hämoglobin binden, **dadurch gekennzeichnet,** daß man ein Immunogen der allgemeinen Formel:

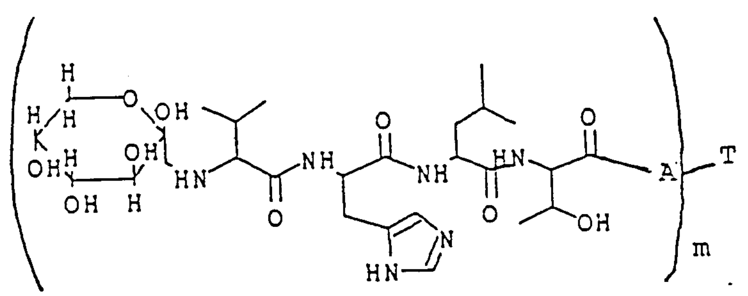

worin m 1 bis zu einer Zahl, die 25 % des Gewichtes des Trägerproteins an Haptenspacergruppen entspricht, bedeutet, T ein Trägerprotein bedeutet und A einen Spacer darstellt, der eine Kettenlänge von 10 bis 20 Atomen aufweist mit der folgenden Formel II.

$$-HN-\underset{\displaystyle COOH}{\underset{|}{CH}}-(CH_2)_n-X-B-$$

worin X S oder NH bedeutet, n 1 bis 4 bedeutet und B einen organischen Rest bedeutet, der eine Succinimidgruppe enthält, in einen zur Antikörperbildung befähigten Organismus injiziert und dann in an sich bekannter Weise die Antikörper gewinnt.

**6.** Verfahren nach Anspruch 5, **dadurch gekennzeichnet,** daß man als Immunogen ein Immunogen gemäß einem der Ansprüche 2 bis 4 verwendet.

**7.** Verwendung der gemäß dem Verfahren nach Anspruch 5 oder 6 erhaltenen Antikörper zur extrakorporalen Bestimmung von $HbA_{1c}$ in Körperflüssigkeiten.

**8.** Zellinie ECACC 87120801.

**9.** Zellinie ECACC 88122302.

**10.** Zellinie ECACC 88122301.

**Patentansprüche für folgende Vertragsstaaten : ES, GR**

**1.** Verfahren zur Gewinnung von Antikörpern, die spezifisch glykosyliertes Hämoglobulin binden,
**dadurch gekennzeichnet,**
daß man ein Immunogen der allgemeinen Formel:

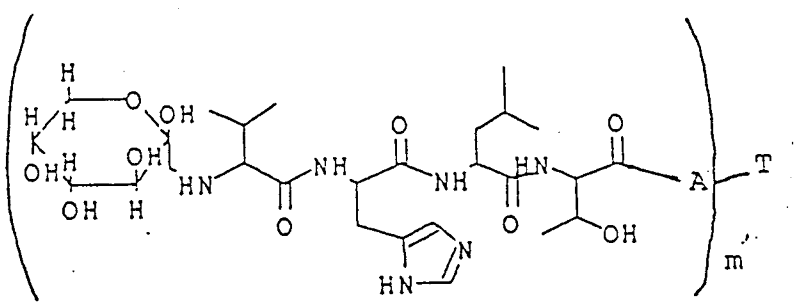

worin m 1 bis zu einer Zahl, die 25 % des Gewichtes des Trägerproteins an Haptenspacergruppen entspricht, bedeutet, T ein Trägerprotein bedeutet und A einen Spacer darstellt, der eine Kettenlänge von 10 bis 20 Atomen aufweist mit der folgenden Formel II

$$-HN-\underset{\displaystyle COOH}{\underset{|}{CH}}-(CH_2)_n-X-B-$$

worin X S oder NH bedeutet, n 1 bis 4 bedeutet und B einen organischen Rest bedeutet, der eine Succinimidgruppe enthält, in einen zur Antikörperbildung befähigten Organismus injiziert und dann die Antikörper aus dem Organismus gewinnt.

**2.** Verfahren nach Anspruch 1,
**dadurch gekennzeichnet,**
daß der Rest A eine Kettenlänge von 12 bis 18 Atomen aufweist.

**3.** Verfahren nach Anspruch 1 oder 2,
**dadurch gekennzeichnet,**
daß m 3 bis 20 bedeutet.

4. Verfahren nach einem der vorhergehenden Ansprüche, **dadurch gekennzeichnet,** daß B einen Succinimidyl-Hexanoylrest darstellt.

5. Verfahren zur Bestimmung von $HbA_{1c}$ in Körperflüssigkeiten, **dadurch gekennzeichnet,** daß man einen Antikörper, der nach einem der Ansprüche 1 bis 4 erhalten wurde, im Rahmen eines Immunoassays einsetzt.

## Claims

**Claims for the following Contracting States : AT, BE, CH, DE, FR, GB, IT, LI, LU, NL, SE**

1. Immunogen for the obtaining of $HbA_{1c}$-specific antibodies with the general formula:-

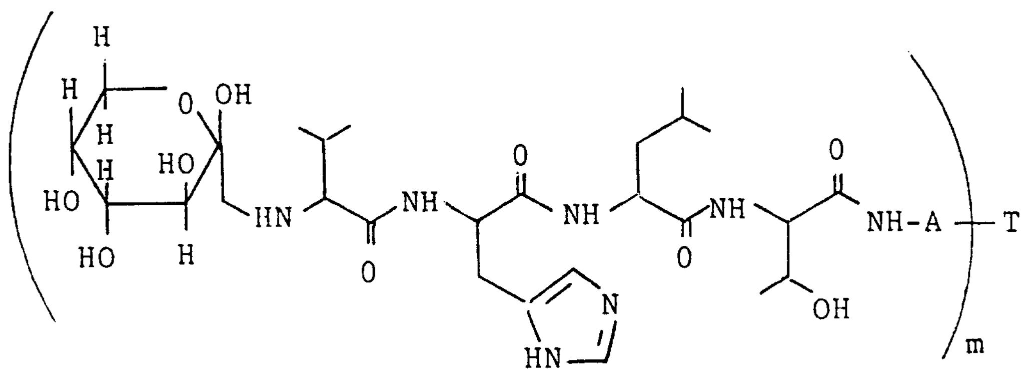

wherein m signifies 1 to a number which corresponds to 25% of the weight of the carrier protein on hapten spacer groups, T signifies a carrier protein and A represents a spacer which has a chain length of 10 to 20 atoms with the following formula II

$$-HN-\underset{COOH}{CH}-(CH_2)_n-X-B-$$

wherein X signifies S or NH, n signifies 1 to 4 and B signifies an organic radical which contains a succinimide group.

2. Immunogen according to claim 1, characterised in that the radical A has a chain length of 12 to 18 atoms.

3. Immunogen according to claim 1 or 2, characterised in that m signifies 3 to 20.

4. Immunogen according to one of the preceding claims, characterised in that B represents a succinimidylhexanoyl radical.

5. Process for the obtaining of antibodies which specifically bind glycosylated haemoglobin, characterised in that one injects an immunogen of the general formula

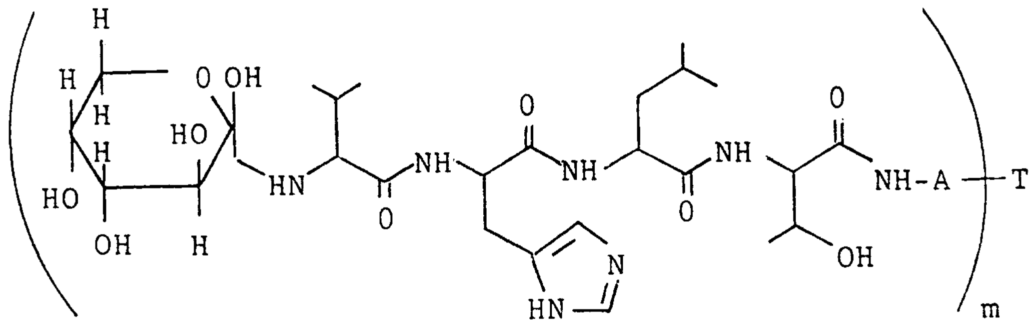

wherein m signifies 1 to a number which corresponds to 25% of the weight of the carrier protein on hapten spacer groups, T signifies a carrier protein and A represents a spacer which has a chain length of 10 to 20 atoms with the following formula II

$$-HN-\underset{COOH}{\underset{|}{CH}}-(CH_2)_n-X-B-$$

wherein X signifies S or NH, n signifies 1 to 4 and B signifies an organic radical which contains a succinimide group, into an organism capable of antibody formation and then obtains the antibodies in per se known manner.

6. Process according to claim 5, characterised in that, as immunogen, one uses an immunogen according to one of claims 2 to 4.

7. Use of antibodies obtained according to the process according to claim 5 or 6 for the extracorporeal determination of $HbA_{1c}$ in body fluids.

8. Cell line ECACC 87120801.

9. Cell line ECACC 88122302.

10. Cell line ECACC 88122301.

**Claims for the following Contracting States : ES, GR**

1. Process for the obtaining of antibodies which specifically bind glycosylated haemoglobin, characterised in that one injects an immunogen of the general formula

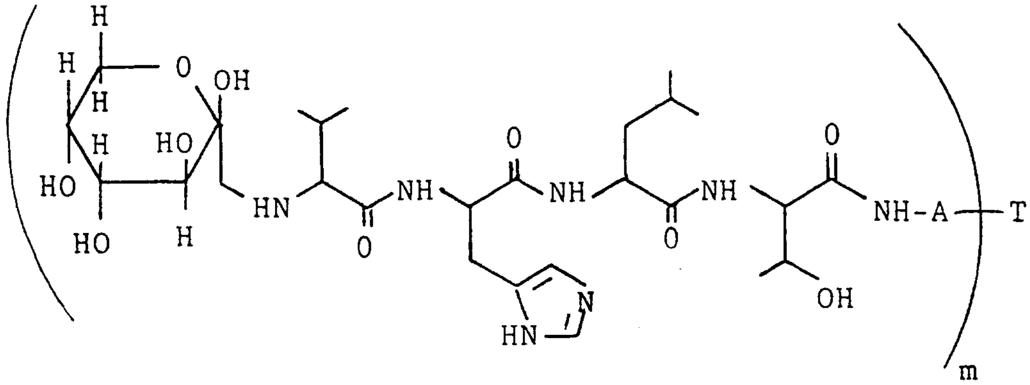

wherein m signifies 1 to a number which corresponds to 25% of the weight of the carrier protein on hapten spacer groups, T signifies a carrier protein and A represents a spacer which has a chain length of 10 to 20 atoms with the following formula II

$$-HN-\underset{\displaystyle COOH}{\underset{|}{CH}}-(CH_2)_n-X-B-$$

wherein X signifies S or NH, n signifies 1 to 4 and B signifies an organic radical which contains a succinimide group, into an organism capable of antibody formation and then obtains the antibodies in per se known manner.

2. Process according to claim 1, characterised in that the radical A has a chain length of 12 to 18 atoms.

3. Process according to claim 1 or 2, characterised in that m signifies 3 to 20.

4. Process according to one of the preceding claims, characterised in that B represents a succinimidylhexanoyl radical.

5. Process for the determination of $HbA_{1c}$ in body fluids, characterised in that one uses an antibody which has been obtained according to one of claims 1 to 4 in the scope of an immunoassay.

**Revendications**

**Revendications pour les Etats contractants suivants : AT, BE, CH, DE, FR, GB, IT, LI, LU, NL, SE**

1. Immunogène pour l'obtention d'anticorps spécifiques de $HbA_{1C}$ de formule générale I:

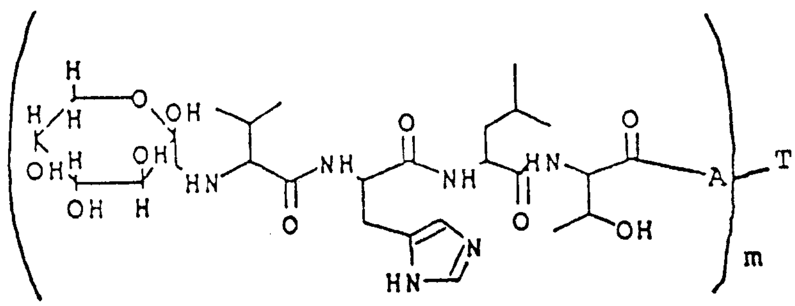

dans laquelle m représente un nombre compris entre 1 et un nombre qui correspond à 25% du poids de la protéine porteuse en groupes espaceurs d'haptène, T représente une protéine porteuse et A représente un espaceur qui présente une longueur de chaîne de 10 à 20 atomes et qui répond à la formule générale II suivante:

$$-HN-\underset{\displaystyle COOH}{\underset{|}{CH}}-(CH_2)_n-X-B-$$

dans laquelle X représente S ou NH, n représente 1 à 4 et B représente un reste organique qui contient un groupe succinimide.

2. Immunogène salon la revendication 1, caractérisé en ce que le reste A présente une longueur de chaîne de 12 à 18 atomes.

3. Immunogène selon la revendication 1 ou 2, caractérisé en ce que m représente 3 à 20.

4. Immunogène selon l'une des revendications précédentes, caractérisé en ce que B représente un reste succinimidyl-hexanoyle.

5. Procédé d'obtention d'anticorps qui fixent spécifiquement l'hémoglobine glycosylée, caractérisé en ce que l'on injecte dans un organisme capable de former des anticorps un immunogène de formule générale:

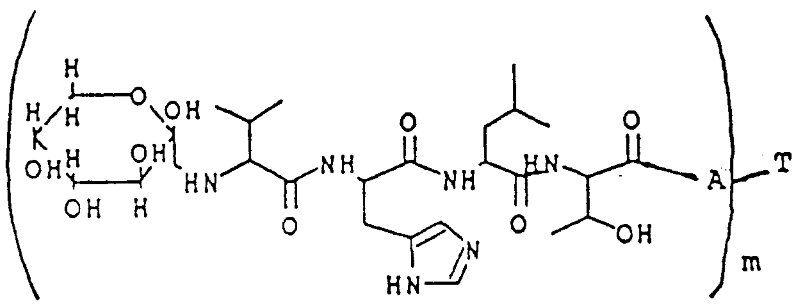

dans laquelle m représente un nombre compris entre 1 et un nombre qui correspond à 25% du poids de la protéine porteuse en groupes espaceurs d'haptène, T représente une protéine porteuse et A représente un espaceur qui présente une longueur de chaîne de 10 à 20 atomes et qui répond à la formule générale II suivante:

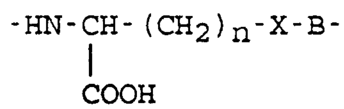

dans laquelle X représente S ou NH, n représente 1 à 4 et B représente un reste organique qui contient un groupe succinimide, puis l'on récupère les anticorps de manière connue en soi.

6. Procédé selon la revendication 5, caractérisé en ce que l'on utilise comme immunogène un immunogène selon l'une des revendications 2 à 4.

7. Utilisation des anticorps obtenus par le procédé selon la revendication 5 ou 6 pour la détermination extracorporelle de $HbA_{1c}$ dans les fluides corporels.

8. Lignée cellulaire ECACC 87120801.

9. Lignée cellulaire ECACC 88122302.

10. Lignée cellulaire ECACC 88122301.

**Revendications pour les Etats contractants suivants : ES, GR**

1. Procédé d'obtention d'anticorps qui fixent spécifiquement l'hémoglobine glycosylée, caractérisé en ce que l'on injecte dans un organisme capable de former des anticorps un immunogène de formule générale:

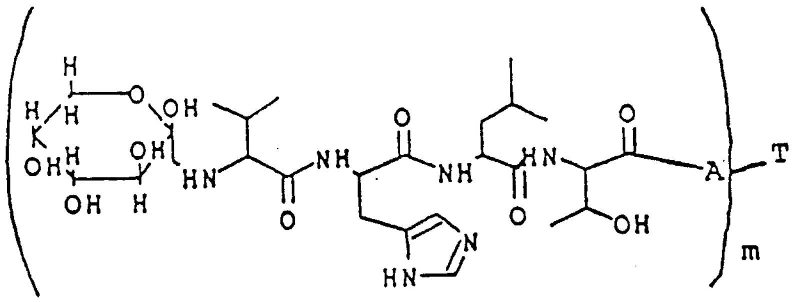

dans laquelle m représente un nombre compris entre 1 et un nombre qui correspond à 25% du poids de la protéine porteuse en groupes espaceurs d'haptène, T représente une protéine porteuse et A représente un espaceur qui présente une longueur de chaîne de 10 à 20 atomes et qui répond à la formule générale II suivante:

$$-HN-\underset{\displaystyle COOH}{\underset{|}{CH}}-(CH_2)_n-X-B-$$

dais laquelle X représente S ou NH, n représente 1 à 4 et B représente un reste organique qui contient un groupe succinimide, puis l'on récupère les anticorps à partir de l'organisme.

2. Procédé selon la revendication 1, caractérisé en ce que le reste A présente une longueur de chaîne de 12 à 18 atomes.

3. Procédé selon la revendication 1 ou 2, caractérisé en ce que m représente 3 à 20.

4. Procédé selon l'une des revendications précédentes, caractérisé en ce que B représente un reste succinimidylhexanoyle.

5. Procédé de détermination de $HbA_{1c}$ dans les fluides corporels, caractérisé en ce que l'on utilise dans le cadre d'une immuno-analyse un anticorps qui a été obtenu selon l'une des revendications 1 à 4.

Fig.1

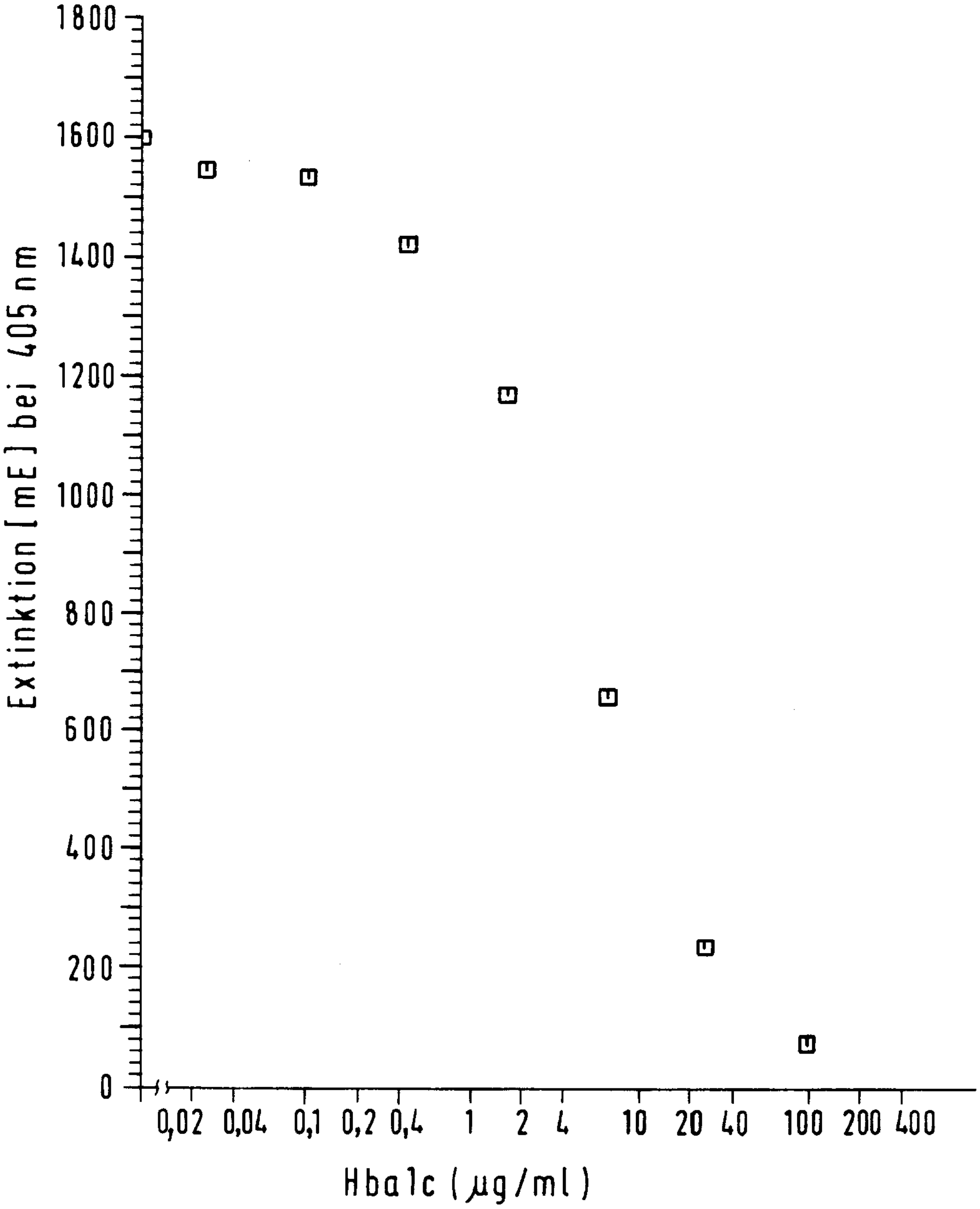
1800
1600
1400
1200
1000
800
600
400
200
0
Extinktion [mE] bei 405 nm
0,02 0,04 0,1 0,2 0,4 1 2 4 10 20 40 100 200 400
Hba1c (µg/ml)

Fig. 2

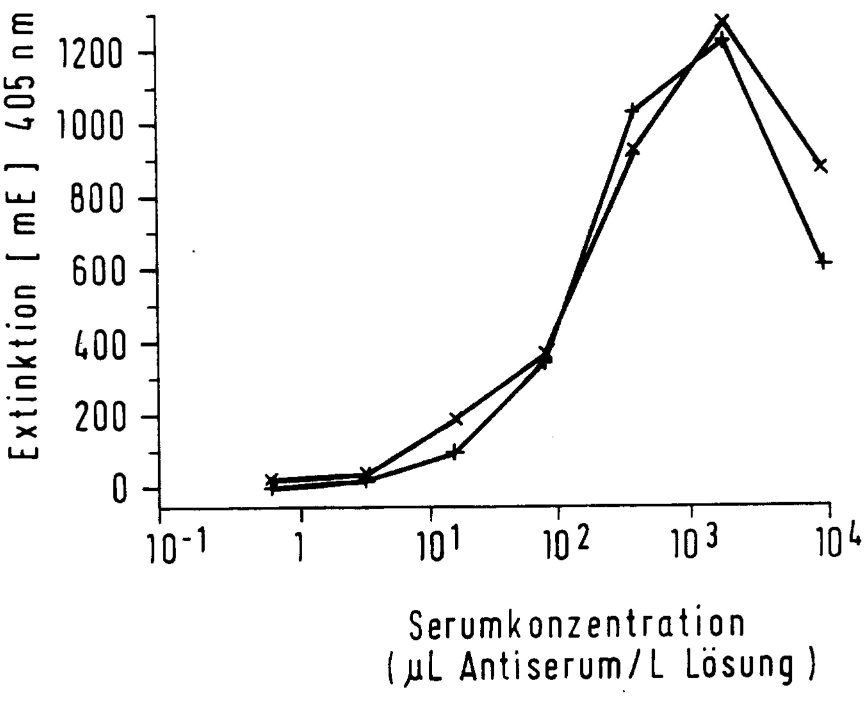
Extinktion [ mE ] 405 nm
1200
1000
800
600
400
200
0
10-1
1
10 1
10 2
10 3
10 4
Serumkonzentration
( µL Antiserum/L Lösung )

Fig. 3

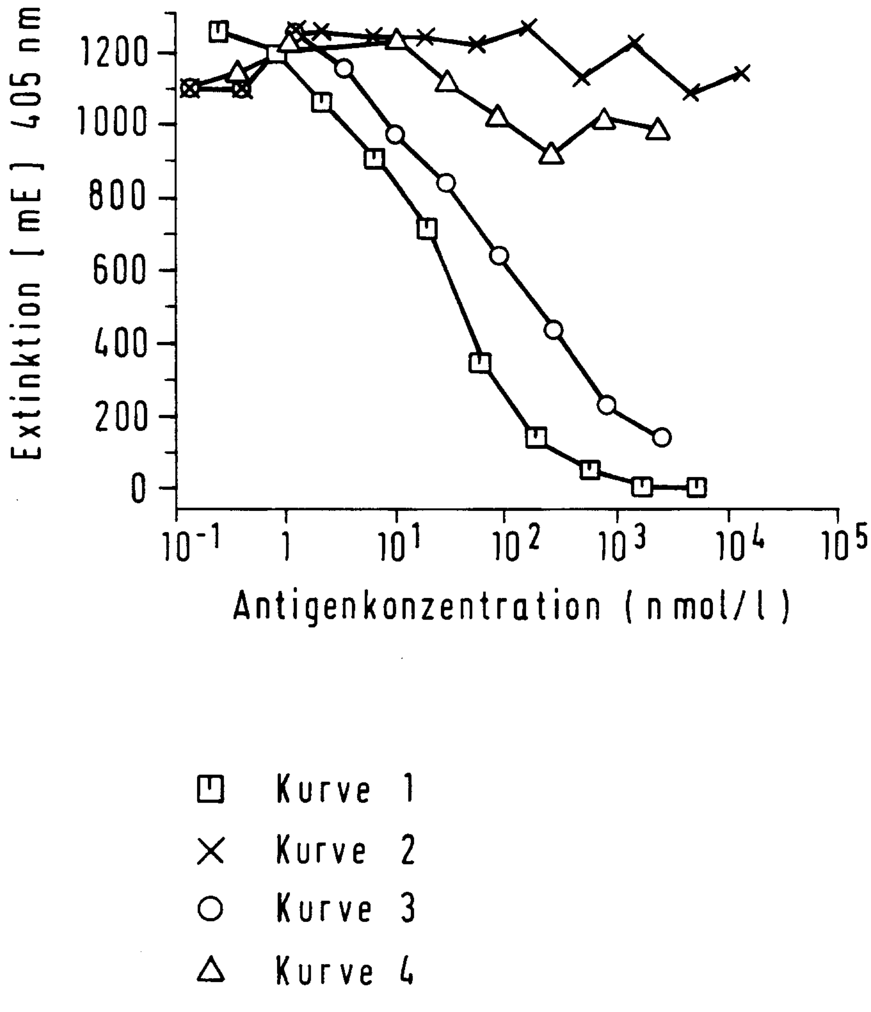
Extinktion [ mE ] 405 nm
1200
1000
800
600
400
200
0
$10^{-1}$
1
$10^1$
$10^2$
$10^3$
$10^4$
$10^5$
Antigenkonzentration ( n mol/l )
Kurve 1
Kurve 2
Kurve 3
Kurve 4